# EUROPEAN PATENT SPECIFICATION

(11) **EP 3 801 455 B1**
(45) Date of publication and mention of the grant of the patent: **21.05.2025**
(21) Application number: 19727397.2
(22) Date of filing: 29.05.2019
(51) Int. Cl.: A61K 31/19, A61K 31/194, A61K 31/60, A61K 33/30, A61K 9/00, A61P 31/10, A61P 17/00, A61P 31/12

(54) **MILD COMPOSITION FOR USE IN TOPICAL TREATMENT OF SKIN AND NAIL DISORDERS CAUSED BY VIRUS AND FUNGUS**
MILDE ZUSAMMENSETZUNG ZUR VERWENDUNG IN DER TOPISCHEN BEHANDLUNG VON DURCH EINEN VIRUS UND PILZ VERURSACHTEN HAUT- UND NAGELSTÖRUNGEN
COMPOSITION DOUCE DESTINÉE À ÊTRE UTILISÉE DANS LE TRAITEMENT TOPIQUE DE TROUBLES DE LA PEAU ET DES ONGLES CAUSÉS PAR UN VIRUS ET DES CHAMPIGNONS

(30) Priority: 30.05.2018 EP 18175098
(43) Date of publication of application: 14.04.2021
(73) Proprietor: Eviderm Institute AB, 170 77 Solna (SE)
(72) Inventor: LODÉN, Marie, 170 77 SOLNA (SE); SWAHN, Britt-Marie, 152 52 SÖDERTÄlJE (SE)
(74) Representative: Brann AB
(86) International application number: PCT/EP2019/063934
(87) International publication number: WO 2019/229114

(56) References cited:
- WO-A2-2011/059324
- US-A1- 2006 003 969
- US-A1- 2011 256 249
- JOE A. KHATTAR ET AL: "Topical zinc oxide vs. salicylic acid?lactic acid combination in the treatment of warts", INTERNATIONAL JOURNAL OF DERMATOLOGY, vol. 46, no. 4, 1 April 2007 (2007-04-01), UK, pages 427 - 430, XP055521838, ISSN: 0011-9059, DOI: 10.1111/j.1365-4632.2006.03138.x
- SHARQUIE K E; KHORSHEED A A; AL-NUAIMY A A: "Topical zinc sulphate solution for treatment of viral warts", SAUDI MEDICAL JOURNAL2007SA, vol. 28, no. 9, 2007, pages 1418 - 1421, XP009509460

## Description

### Field of the invention

The present invention relates to a composition comprising an alpha hydroxy acid selected from the group consisting of malic, lactic and glycolic acids, and combinations thereof, a water-soluble zinc salt, e.g. zinc sulphate, and methyl salicylate for use in the treatment of skin and nail disorders caused by virus and fungus in mammals, especially in humans, by topical administration of the formulation on the affected area, as well as a kit comprising the formulation and an applicator, an applicator connected to a container holding the composition. A method of treating the disorders in a subject using the formulation is disclosed herein. It is noted that any references to methods of treatment refer to the compositions of the present invention for use in a method for treatment of the human (or animal) body by therapy.

### Background art

A cutaneous wart is generally a small, rough growth, typically on a human's hands or feet. They are caused by a viral infection, specifically by one of the many types of human papilloma viruses (HPV). It is possible to get warts from others, thus they are contagious and usually enter the body in an area of broken skin. A range of types of wart have been identified, varying in shape and site affected, as well as the type of human papilloma virus involved.

Common warts have a characteristic appearance under the microscope. They exhibit a thickening of the stratum corneum (hyperkeratosis), thickening of the stratum spinosum (acanthosis), thickening of the stratum granulosum, rete ridge elongation, and large blood vessels at the dermoepidermal junction. Most warts, including flat warts and plantar warts, display some type of skin abnormality, such as hyperkeratotic surfaces.

Treatment of warts is typically painful, prolonged and can cause scarring. The treatments of warts, which presently exist, mainly destroy the outer layer of the skin on which the wart is growing. This can be both painful and unpleasant for the patients, especially in children. The chemical preparations that are commonly used today contain acids such as salicylic acid, trichloroacetic acid or formic acid in different concentrations and are strongly corrosive. The skin is strongly challenged by these applications and inflammations are often the result. Sharquie et al. ("Topical zinc sulphate solution for treatment of viral warts", SaudiMedical Journal 2007, 28(9), 1418-21) describe the efficacy and safety of topical zinc sulphate solution in the treatment of plane warts. Sharquie et al. conclude that 10% topical zinc sulphate is an effective, non-costly, new therapy for treatment of plane warts. Topical zinc sulphate solution was however found to be ineffective in patients with common warts, probably due to thick hyperkeratotic surface that prevent penetration of the drug.

Khattar et al. ("Topical zinc oxide vs. salicylic acid-lactic acid combination in the treatment of warts", International Journal of Dermatology 2007, 46(4), 427-30) compares the cure rates of warts treated with 20% zinc oxide ointment vs. those treated with a 15% salicylic acid and 15% lactic acid combination. The patients were followed up for 3 months or until cure, whichever came first. Under the heading "Discussion" Khattar et al. state that zinc oxide 20% ointment seems promising and is comparable with 15% salicylic acid combined with 15% lactic acid ointment in the treatment of warts. Under the heading "Conclusion" Khattar et al. find that zinc oxide is simple to apply and painless, and therefore may be promising for the treatment of children.

Fungal infection of the toenails or fingernails is caused by a fungal microbe that invades the nail bed. Fungal nail infection is also termed onychomycosis and tinea unguium. Fungal nail infection causes fingernails or toenails to thicken and discolor. Treatment is aimed at eradication of the causative organism, such as for example trichohopytum rubrum, and promotes a healthy appearance of the nail. The major challenge with treatment of onychomycosis is the thick nail plate which protects the fungus and allows them to survive in the skin.

It is an object of the present invention to provide an alternative and less painful, yet effective, treatment for removal of warts and nail fungus. Reduced pain is especially important for use on children.

### Summary of the invention

According to the invention the above object has been achieved by a composition comprising: one or more alpha hydroxy acids selected from the group consisting of lactic acid, malic acid, and glycolic acid; a water soluble zinc salt; and methyl salicylate.

In one aspect the present invention thus relates to a composition comprising: one or more alpha hydroxy acids selected from the group consisting of lactic acid, malic acid, and glycolic acid in a total amount of from 50 to 80% by weight; from 0.1 to 10% by weight of methyl salicylate; together with a water soluble zinc salt in an amount, calculated as Zn, of from 0.2 to 4% by weight of the formulation.

According to the invention a combination of the above constituents has been found to form a mild and effective wart removal composition within the above ranges of amounts of the constituents. The inventive composition avoids the use of corrosive acids, such as e.g. formic acid, and also avoids the use of painful freezing technique.

It is believed that the combined efficacy and mildness of the inventive composition is derived from the combination of an alpha hydroxy acid, Zn ions and methyl salicylate which unexpectedly enhances the bioavailability of the mixture and removes the warts and the fungus beneath the nail plate, without inducing pain and irritation.

The composition of the invention can be used in treating warts, wherein the inventive composition is applied topically to an area of affected skin. Due to the reduced pain associated with the method disclosed herein, the method is especially well-suited for use on children.

The inventive composition is effective in the treatment of warts caused by human papilloma virus.

Fungi are known to be sensitive to acids, and the inventive composition is also useful for the treatment of fungal infections of the toenails or fingernails.

The inventive composition can be used in treating fungal infections of the toenails or fingernails, wherein the inventive composition is applied topically to an area of an affected nail.

In another aspect the present invention relates to an applicator attached to a container containing the inventive composition, such as a pen applicator, which applicator can be used for topical administration of the composition on an affected skin or nail area for the treatment of warts or other viable infections, such as fungus in the nails.

It is believed that the inventive composition also will be effective in the treatment of fungal infection on the skin of feet.

Further aspects and advantages will become evident from the detailed description and appended claims.

### Detailed description of the invention

The alpha hydroxy acids used according to the present invention have been found by the present inventors to provide a milder alternative to the corrosive acids used in prior art wart removal formulations.

According to the invention, zinc ions have surprisingly been found to make the alpha hydroxy acid(s), which are used in the inventive composition, more tolerable and less irritating to the skin, thus making the inventive composition even milder, and also allowing for higher concentrations of the alpha hydroxy acid(s) to be used in the inventive formulation.

Zinc is known to have an antiviral effect. It is believed that the one or more alpha hydroxy acids of the invention will potentiate said effect of the zinc, such as possibly by enhancing penetration of zinc into the skin. Such enhanced penetration effect of zinc could improve the effectiveness of the inventive composition in treating a viral infection in the skin or nail. An especially preferred alpha hydroxy acid in this regard is glycolic acid.

In the inventive composition methyl salicylate is believed to further enhance penetration of the alpha hydroxy acid and zinc into the skin.

Zinc ions also seem to have a similar effect on methyl salicylate, thus also making the methyl salicylate, which is used in the inventive composition, more tolerable and less irritating to the skin.

Within the inventive range of zinc concentration (0.2-4) there seems to be a dose-response relationship. A preferred range of zinc is from 0.2-3% by weight, and more typically from 0.5-3% by weight.

The present invention enables accomplishing a composition, devoid of strong corrosive acids, for the treatment of warts and onychomycosis.

Glycolic acid is used in dermatological treatments depending on its exfoliant capacity. Glycolic acid reacts with the upper layer of the epidermis, weakening the binding properties of the lipids that hold the dead skin cells together. This allows the stratum corneum to be exfoliated, thereby exposing live skin cells. Alpha hydroxy acids also reduce the number of desmosomes and tonofilaments. Other acids that can be used due to their exfoliant capacity are lactic acid and malic acid. Glycolic acid is however generally preferred according to the invention.

It is believed that an exfoliant, such as glycolic acid, may facilitate the solubility of the zinc ions and accelerate the removal of virus infected stratum corneum. For this purpose higher concentrations of glycolic acid are preferred.

In its most generic embodiment, the inventive composition contains a water soluble zinc salt, e.g. zinc sulphate, methyl salicylate, and an alpha hydroxy acid, selected from glycolic acid, lactic acid and malic acid, and combinations thereof.

The zinc ions of the inventive formulation can be generated from zinc salts selected from the group consisting of zinc sulphate, zinc chloride, zinc acetate, zinc glycolate, zinc gluconate, zinc carbonate, zinc oxide, zinc oleate, zinc stearate, zinc propionate, zinc salicylate, zinc lactate, zinc coceth sulphate, zinc citrate, zinc ascorbate and zinc glutamate. Preferred zinc salts are zinc sulphate, zinc chloride and zinc acetate. Especially preferred is zinc sulphate.

Methyl salicylate is considered to be a permeability enhancer. This enhancer acts as a solvent and interacts with and modifies the lipid domains of the stratum corneum and could thus further increase the permeability of zinc ions and the acid. The inventive combination of alpha hydroxy acid and methyl salicylate could thus serve as a powerful penetration enhancer for the active mixture.

Moreover, methyl salicylate has a pleasant smell and will also increase the agreeability of the inventive formulation.

Methyl salicylate is preferably used in an amount within the range of 0.2-10%, and more typically in an amount of 0.5-10% by weight of the composition.

Methyl salicylate will however typically not be soluble in a composition comprising for example merely glycolic acid and zinc sulphate. Accordingly, a solubiliser may be required for dissolving the methyl salicylate. The composition according to the invention thus preferably includes a solubiliser for the methyl salicylate. Different solubilisers, such as e.g. ethoxylated fatty alcohols, for example Oleth-20, can be used to solubilise methyl salicylate in the inventive formulation.

In the case of Oleth-20, it was discovered that a very narrow and bell-shaped concentration the solubiliser created clear solutions wherein the methyl salicylate had been dissolved. A suitable amount of the solubilizer is about twice the amount of methyl salicylate. Accordingly, for the inventive range of methyl salicylate of from 0.1 to 10% by weight, the preferred corresponding amount of solubilizer is from 0.2 to 20% by weight, and for the preferred range of from 0.2-10% of methyl salicylate, the preferred corresponding amount of solubilizer is from 0.4 to 20% by weight etc. An especially preferred solubilizer is Oleth-20.

It was discovered that the inventive composition comprising methyl salicylate displayed a deeper effect on skin compared to a composition devoid of methyl salicylate. A deeper effect will also occur on the nail bed due to enhanced penetration of the actives.

In certain preferred embodiments methyl salicylate in an amount of from 2 to 5% w/w may be solubilised with Oleth-20 in an amount of from 4% to 10% w/w in a formulation comprising zinc sulphate (ZnSO₄x7H₂O) in an amount of from 1.0 % to 2.5 % w/w based on Zn, and glycolic acid of from 55 to 65% w/w.

The inventive composition can be provided as a kit comprising the composition and a device for topical application thereof, i.e. an applicator. Suitable applicators are known in the art, such as, e.g. a cotton swab, cotton tip, spatula, sponge applicator, brush and the like etc.

The applicator can be attached to a container holding the composition and capable of providing composition contained therein to the applicator. Suitable applicators are known in the art, such as e.g pen-like applicators, dispensing pens, and topical metered dosing dispensers. Accordingly, the inventive composition can for example conveniently be provided in a pen applicator for the topical administration of the composition from a tip of the pen to an affected skin area for the treatment of warts. The tip of such applicator, from which tip the inventive composition is applied to an affected area, can for example be felt, a brush, or a roller ball.

It is also conceivable to apply the inventive composition by spraying, such as e.g. to the skin of feet for treating fungal infections on the feet. The inventive composition could thus be provided contained in a dispenser for dispensing an aearosol.

### Examples

The following examples are included to explain the principles of the present invention and are not intended to be understood as limiting the claimed scope. It will be apparent to those of ordinary skill in the art that modifications, including but not limited to, variations in amount, ingredients, mixing and application technique may be made without departing from the principles and concepts described herein.

All chemicals used herein are used as acquired from the vendors.

### Example 1

The formulations in Example 1 were manufactured by mixing the components specified in table 1 below. The mixture was then heated on a water bath until a clear solution was obtained. The appearance of the different formulations was visually examined directly, after two hours, and after 4 days. The read-outs are included in the table 1.

**Table 1. The influence of different concentrations of Oleth-20 and zinc sulphate on the clarity of the formulation.**

| | 1A | 1B | 1C | 1D | 1E |
|---|---|---|---|---|---|
| Component | w% | w% | w% | w% | w% |
| Glycolic acid | 65 | 65 | 65 | 65 | 65 |
| Methyl salicylate | 5 | 5 | 5 | 5 | 5 |
| Oleth-20 | 20 | 15 | 10 | 8 | 5 |
| ZnSO₄x7H₂O* | 5 | 5 | 5 | 5 | 5 |
| Water | 5 | 10 | 15 | 17 | 20 |
| Total | 100 | 100 | 100 | 100 | 100 |
| Appearance 0 h | thick-clear | clear | clear | clear | opalescent |
| 1-2 h | solid | clear | clear | opalescent | opalescent |
| ~4 days | solid | clear | clear | opalescent | opalescent |

**Table 1 (continued).**

| | 1G | 1H | 1K |
|---|---|---|---|
| Component | w% | w% | w% |
| Glycolic acid | 65 | 65 | 65 |
| Methyl salicylate | 5 | 5 | 5 |
| Oleth-20 | 10 | 10 | 8 |
| ZnSO₄x7H₂O* | 7 | 3 | 7 |
| Water | 13 | 17 | 15 |
| Total | 100 | 100 | 100 |
| Appearance 0 h | clear | clear | clear |
| 1-2 h | clear | clear | clear-opal. |
| ~4 days | clear | clear | opalescent |

| | | | |
|---|---|---|---|
| * Given as added amount of Zn-salt: 5 % by weight of the Zn-salt corresponds to 1.1 % by weight of Zn; 3 % by weight of the Zn-salt corresponds to 0.7 % by weight of Zn; and, 7 % by weight of the Zn-salt corresponds to 1.6 % by weight of Zn. | | | |

From these experiments it was discovered that a very narrow and bell-shaped concentration of Oleth-20 produced clear solutions. Smaller variations in the concentration of zinc did not alter the solubility.

### Example 2

In order to confirm the postulated increased skin permeability by the combination of the alpha hydroxy acid and methyl salicylate an objective measurement protocol was used. The measurements were performed with a DermaLab^{®} Combo (Cortex Technology, Hadsund, Denmark). Measurement of skin color is based on an active color detecting chip where illumination is provided by white LEDs and the measure of erythema corresponds to the redness (hemoglobin) of the skin. The target area for measurement is 7 mm in diameter. A surrogate marker, 0.4% methyl nicotinate in ethanol, was used since it causes redness of the skin when it penetrates the skin. Increased redness means that a larger amount of methyl nicotinate has penetrated the skin and thus the uptake has increased. As uptake of the active inventive complex of Zn ion, acid, and methyl salicylate cannot be measured in this way methyl nicotinate was used as surrogate marker. The formulations in Table 2 are thus not formulations of the invention.

**Table 2 Penetration enhancement of methyl nicotinate induced by methyl salicylate.**

| | 2A (comparative) | 2B (comparative) | 2C (comparative) |
|---|---|---|---|
| Component | w% | w% | w% |
| Glycolic acid | 59.5 | 59.5 | 59.5 |
| Methyl salicylate | 5.0 | - | - |
| Oleth-20 | 10.0 | 10.0 | - |
| Water | 25.5 | 30.5 | 40.5 |
| Appearance 1 hour | clear | clear | clear |
| Appearance 1 month | clear | clear | clear |

| Time after methyl nicotinate | Read-out from DermaLab^{®} Combo. (erythema) | | |
|---|---|---|---|
| 0 minutes | 10.5 | 10.3 | 10.8 |
| 5 | 10.8 | 10.9 | 10.8 |
| 10 | 12.7 | 12.1 | 12.5 |
| 12 | 12.9 | 13.1 | 12.5 |
| 15 | 14.9 | 13.5 | 13.6 |
| 25 | 15.1 | 13.4 | 13.6 |
| 40 | 14.6 | 13.2 | 12.2 |
| Difference at t=0, t=25 min | 4.6 | 3.1 | 2.8 |

The formulations in Table 2 were manufactured by mixing the components set forth in the upper part of table 2. The mixture was then heated on a water bath until a clear solution was obtained. The appearance of the different formulations was visually examined after one hour and after 1 month. The read-outs are included in the lower part of table 2.

The formulations 2A, 2B and 2C were evaluated on intact skin after topical administration of 2 µL of the different formulations shown in table 2. After 50 minutes 2 µL of methyl nicotinate (0.4%) was administrated on the same spot as the formulations 2A, 2B and 2C. The colour-redness (erythema) was measured at different points of time by DermaLab^{®} Combo and the results are shown in the lower part of table 2. The increase in redness was largest for the formulation containing both methyl salicylate and Oleth-20, with a maximum difference 4.6 for formulation 2A, 3.1 for 2B and 2.8 for 2C.

It was concluded that a formulation comprising glycolic acid, methyl salicylate and Oleth-20 has advantages for the penetration of the skin compared to a formulation devoid of methyl salicylate and Oleth-20.

### Example 3

The formulations in Example 3 were manufactured by mixing the components set forth in the upper part of table 3. The mixture was then heated on a water bath until a clear solution was obtained. The appearance of the different formulations was visually examined after one hour. The read-outs are included in the lower part of table 3. Formulations 3B, 3D, and 3E are only comparative. Only 3C is inventive.

**Table 3. The influence of each component on the degree of erythema.**

| | 3B (comparative) | 3C | 3D (comparative) | 3E (comparative) |
|---|---|---|---|---|
| Component | w% | w% | w% | w% |
| Glycolic acid | 0 | 65 | 65 | 65 |
| Methyl salicylate | 5 | 5 | 0 | 5 |
| Oleth-20 | 10 | 10 | 0 | 10 |
| ZnSO₄x7H₂O* | 5 | 5 | 5 | 0 |
| Water | 80 | 15 | 30 | 20 |
| Total | 100 | 100 | 100 | 100 |
| Appearance 1 h | opalescent | clear | clear | clear |
| Effect on skin Occlusion (40 µL for 6 h) | | | | |
| Visible read-out 2 min | - | + | - | ++ |
| Erythema 2 min | 11 | 14.3 | 13 | 15 |
| Visible read-out 48 hours | - | ++ | + | ++++ |
| Erythema | 11 | 15 | 12 | 21 |
| TEWL | 8 | 23 | 16 | 75 |

| | | | | |
|---|---|---|---|---|
| * Given as added added amount of Zn-salt: 5 % by weight of the Zn-salt corresponds to 1.1 % by weight of Zn. | | | | |

The formulations 3B, 3C, 3D and 3E were evaluated by an occlusion study on intact skin where 40 µL of the formulations was added to patches (Finn-chambers) and then attached to the underarm for 6 hours. The effect on skin was evaluated visibly directly after removal of the patches (after 2 minutes) and after 48 hours according to a five grade scale where (-) means no visible effect on skin, (+) indicate a minor visible effect on skin and (++++) a major mark on the skin. The results are displayed in the lower part of Table 3.

The measurements of erythema and TransEpidermal Water Loss (TEWL) were performed with a DermaLab^{®} Combo (Cortex Technology, Hadsund, Denmark). Measurement of skin color is based on an active color detecting chip where illumination is provided by white LEDs and the measure of erythema corresponds to the redness (hemoglobin). TEWL is a measure of skin barrier function to water and is made with an open-chamber with two combined humidity/temperature sensors mounted in a cylindrical diffusion chamber (10 mm in diameter). After application of the probe onto the skin, the TEWL value is recorded into the computer until equilibrium. This value is used for further calculations. Erythema and TEWL values for the different time points are reported in the Table 3.

It was concluded that the comparative mixture (3B) containing methyl salicylate and no glycolic acid did not produce any effect on the read-outs, and the comparative formulation (3D) containing glycolic acid and no methyl salicylate caused only a minor effect. Surprisingly it was found that the inventive mixture (3C) containing zinc sulphate, glycolic acid, and methyl salicylate produced much less visible and measured effect as compared to the comparative mixture (3E) devoid of zinc sulphate. The results indicate that the inventive formulation containing methyl salicylate and glycolic acid has advantages for the penetration of the skin of zinc ions, thus producing a milder formulation than compared to a formulation devoid of zinc. The inventive formulation 3C was astonishingly discovered to be a new mild and effective treatment of warts.

### Example 4A - effect on warts:

The invention has been proved to be effective with disappearing warts without any reported side effects.

Individuals having warts, on the feet, toes, and/or hands were treated successfully with composition 5B. The treatment until removal of the wart(s) varied from a few days to 4 weeks Older deeper warts generally required a longer period of treatment, while new warts disappeared merely after a shorter treatment.

### Example 4B - effect on onychomycosis:

Woman (59 y) - long standing yellowing and thickening of the nails on the foot. Several treatment-failures with established medicinal products against nail fungus. Within two months of treatment with the inventive formulation 5B successful improvement with normal appearing nail and non-infected nail bed was achieved.

### Example 5

The formulations in example 5 were manufactured by mixing the components in table 5. The mixture was then heated on a water bath until a clear solution was obtained. The appearance of the different formulations was visually examined after one hour. The read-outs are included in the table 5.

**Table 5. The inventive mixture with zinc sulphate gives significantly less irritation compared to a mixture without zinc sulphate.**

| | 5B | | 5D (comparative) | |
|---|---|---|---|---|
| Component | w% | | w% | |
| Glycolic acid | 55 | | 55 | |
| Methyl salicylate | 2 | | 2 | |
| Oleth-20 | 4 | | 4 | |
| ZnSO₄x7H₂O* | 10 | | - | |
| Water | 29 | | 39 | |
| Total | 100 | | 100 | |
| Appearance 1 h | clear | | clear | |
| Occlusion 40 µL, 8 h | Patient1 | Patient2 | Patient1 | Patient2 |
| Read-out 15 min: visible | - | - | ++ | ++ |
| Read-out 48 h: visible | - | - | ++++ | +++ |
| Erythema | 13 | | 23 | |
| TEWL | 8 | | 72 | |

| | | | | |
|---|---|---|---|---|
| * Given as added amount of Zn-salt: 10 % by weight of the Zn-salt corresponds to 2.3 % by weight of Zn. | | | | |

Inventive formulation 5B and comparative formulation 5D were evaluated by an occlusion study on intact skin (on two patients) where 40 µL of the formulations was added to patches (Finn-chambers) and then attached to the underarm for 8 hours. The effect on skin was evaluated visibly directly after removal of the patches (after 15 minutes) and after 48 hours according to a five-grade scale where (-) means no visible effect on skin, (+) indicate a minor visible effect on skin and (++++) a major mark on the skin. The results are displayed in the Table 5.

The measurements of erythema and TEWL were performed with a DermaLab^{®} Combo as described above. Erythema and TEWL values for the different points of time are reported in the Table 5.

It was surprisingly discovered an unpredictably large difference in the measured and visible read-out between the inventive formulation 5B containing zinc sulphate, and the comparative formulation 5D devoid of a water soluble zinc salt, such as zinc sulphate.

The surprising effect of the zinc ions can be seen from 3C vs. 3E, and 5B vs. 5D, respectively. It can also be seen that the higher percentage of zinc, 2.3 %, used in Example 5 produced a larger effect than the lower concentration of 1.1 % used in Example 3.

## Claims

1. A topical composition comprising: an alpha hydroxy acid selected from the group consisting of lactic acid, malic acid, and glycolic acid, and combinations thereof in a total amount of from 50 to 80 % by weight; 0.1 to 10 % by weight of methyl salicylate; and, a water soluble zinc salt in an amount of from 0.2 to 4 % by weight of the formulation calculated as Zn.

2. The composition of claim 1, further comprising a solubiliser in an amount effective for solubilizing the methyl salicylate in the formulation.

3. The composition of claim 2, wherein said solubiliser is an ethoxylated fatty alcohol.

4. The composition of any one of the preceding claims, wherein the total amount of the alpha hydroxy acid is within the range of 50-70 % by weight.

5. The composition of any one of the preceding claims, wherein the amount of methyl salicylate is 0.2-10 % by weight.

6. The composition of any one of the preceding claims, wherein the alpha hydroxy acid is glycolic acid.

7. The composition of any one of the preceding claims, wherein the water soluble zinc salt is one or more members selected from the group consisting of zinc sulphate, zinc chloride, zinc acetate, zinc glycolate, zinc gluconate, zinc carbonate, zinc oxide, zinc oleate, zinc stearate, zinc propionate, zinc salicylate, zinc lactate, zinc coceth sulphate, zinc citrate, zinc ascorbate and zinc glutamate.

8. The composition of claim 7, wherein the water soluble zinc salt is zinc sulphate.

9. The composition according to any one of the preceding claims, wherein the amount by weight of zinc is from 1.0 to 2.5 %, glycolic acid is from 55 to 65 %, and methyl salicylate is from 2 to 5 %, respectively.

10. A kit comprising a container holding the composition of any one of claims 1-9, and an applicator for topical application of the composition.

11. The kit of claim 10, wherein the applicator comprises a felt tip, a brush tip, or a roller ball tip attached to the container, from which container the composition can be delivered to the tip of the applicator.

12. A pen containing the composition of any one of claims 1-9 for the topical administration of the composition from a tip of the pen to an affected skin area for the treatment of warts.

13. The composition of any one of claims 1-9 for use in treating warts wherein the composition is applied to an affected skin area of a subject.

14. The composition of any one of claims 1-9 for use in the treatment of fungal infections of the toenails or fingernails wherein the composition is applied topically to an area of affected nail.

## Patentansprüche

1. Eine topische Zusammensetzung, umfassend: eine Alpha-Hydroxysäure, ausgewählt aus der Gruppe bestehend aus Milchsäure, Äpfelsäure und Glykolsäure und Kombinationen davon in einer Gesamtmenge von 50 bis 80 Gew.-%; 0,1 bis 10 Gew.-% Methylsalicylat; und ein wasserlösliches Zinksalz in einer Menge von 0,2 bis 4 Gew.-% der Formulierung, berechnet als Zn.

2. Die Zusammensetzung nach Anspruch 1, ferner umfassend einen Lösungsvermittler in einer Menge, die zum Löslichmachen des Methylsalicylats in der Formulierung wirksam ist.

3. Die Zusammensetzung nach Anspruch 2, wobei der Lösungsvermittler ein ethoxylierter Fettalkohol ist.

4. Die Zusammensetzung nach einem der vorhergehenden Ansprüche, wobei die Gesamtmenge der Alpha-Hydroxysäure im Bereich von 50 bis 70 Gew.-% liegt.

5. Die Zusammensetzung nach einem der vorhergehenden Ansprüche, wobei die Menge an Methylsalicylat 0,2 bis 10 Gew.-% beträgt.

6. Die Zusammensetzung nach einem der vorhergehenden Ansprüche, wobei die Alpha-Hydroxysäure Glykolsäure ist.

7. Die Zusammensetzung nach einem der vorhergehenden Ansprüche, wobei das wasserlösliche Zinksalz eines oder mehrere Mitglieder, ausgewählt aus der Gruppe bestehend aus Zinksulfat, Zinkchlorid, Zinkacetat, Zinkglykolat, Zinkgluconat, Zinkcarbonat, Zinkoxid, Zinkoleat, Zinkstearat, Zinkpropionat, Zinksalicylat, Zinklactat, Zinkcocethsulfat, Zinkcitrat, Zinkascorbat und Zinkglutamat, ist.

8. Die Zusammensetzung nach Anspruch 7, wobei das wasserlösliche Zinksalz Zinksulfat ist.

9. Die Zusammensetzung nach einem der vorhergehenden Ansprüche, wobei die Gewichtsmenge an Zink 1,0 bis 2,5% beträgt, an Glykolsäure 55 bis 65% beträgt bzw. an Methylsalicylat 2 bis 5% beträgt.

10. Ein Kit, umfassend einen Behälter, der die Zusammensetzung nach einem der Ansprüche 1-9 enthält, und einen Applikator zum topischen Aufbringen der Zusammensetzung.

11. Das Kit nach Anspruch 10, wobei der Applikator eine Filzspitze, eine Pinselspitze oder eine Rollerball-Spitze umfasst, die an dem Behälter angebracht ist, wobei die Zusammensetzung aus dem Behälter der Spitze des Applikators zugeführt werden kann.

12. Ein Stift, der die Zusammensetzung nach einem der Ansprüche 1-9 enthält, zur topischen Verabreichung der Zusammensetzung von einer Spitze des Stiftes auf einen betroffenen Hautbereich zur Behandlung von Warzen.

13. Die Zusammensetzung nach einem der Ansprüche 1-9 zur Verwendung bei der Behandlung von Warzen, wobei die Zusammensetzung auf einen betroffenen Hautbereich einer Person aufgebracht wird.

14. Die Zusammensetzung nach einem der Ansprüche 1-9 zur Verwendung bei der Behandlung von Pilzinfektionen der Zehennägel oder Fingernägel, wobei die Zusammensetzung topisch auf einen Bereich des betroffenen Nagels aufgebracht wird.

## Revendications

1. Composition topique comprenant : un alpha hydroxyacide choisi dans le groupe constitué d'acide lactique, d'acide malique et d'acide glycolique, et leurs combinaisons dans une quantité totale de 50 à 80 % en poids ; de 0,1 à 10 % en poids de salicylate de méthyle ; et, un sel de zinc soluble dans l'eau dans une quantité de 0,2 à 4 % en poids de la formulation calculée en tant que Zn.

2. Composition selon la revendication 1, comprenant en outre un agent solubilisant dans une quantité efficace pour solubiliser le salicylate de méthyle dans la formulation.

3. Composition selon la revendication 2, dans laquelle ledit agent solubilisant est un alcool gras éthoxylé.

4. Composition selon l'une quelconque des revendications précédentes, dans laquelle la quantité totale d'alpha-hydroxyacide est comprise dans la plage de 50 à 70 % en poids.

5. Composition selon l'une quelconque des revendications précédentes, dans laquelle la quantité de salicylate de méthyle est de 0,2 à 10 % en poids.

6. Composition selon l'une quelconque des revendications précédentes, dans laquelle l'alpha-hydroxyacide est de l'acide glycolique.

7. Composition selon l'une quelconque des revendications précédentes, dans laquelle le sel de zinc soluble dans l'eau est un ou plusieurs éléments choisis dans le groupe constitué de sulfate de zinc, chlorure de zinc, acétate de zinc, glycolate de zinc, gluconate de zinc, carbonate de zinc, oxyde de zinc, oléate de zinc, stéarate de zinc, propionate de zinc, salicylate de zinc, lactate de zinc, sulfate de cocéth de zinc, citrate de zinc, ascorbate de zinc et glutamate de zinc.

8. Composition selon la revendication 7, dans laquelle le sel de zinc soluble dans l'eau est du sulfate de zinc.

9. Composition selon l'une quelconque des revendications précédentes, dans laquelle la quantité en poids de zinc est de 1,0 à 2,5 %, l'acide glycolique est de 55 à 65 %, et le salicylate de méthyle est de 2 à 5 %, respectivement.

10. Kit comprenant un récipient contenant la composition selon l'une quelconque des revendications 1 à 9, et un applicateur pour application topique de la composition.

11. Kit selon la revendication 10, dans lequel l'applicateur comprend une pointe en feutre, une pointe à brosse ou une pointe à bille roulante fixée au récipient, à partir duquel la composition peut être distribuée à la pointe de l'applicateur.

12. Stylo contenant la composition selon l'une quelconque des revendications 1 à 9 pour l'administration topique de la composition à partir d'une pointe du stylo sur une zone cutanée affectée pour le traitement de verrues.

13. Composition selon l'une quelconque des revendications 1 à 9 pour une utilisation dans le traitement de verrues, dans laquelle la composition est appliquée sur une zone cutanée affectée d'un sujet.

14. Composition selon l'une quelconque des revendications 1 à 9 pour une utilisation dans le traitement d'infections fongiques des ongles des pieds ou des mains, dans laquelle la composition est appliquée de façon topique sur une zone d'ongle affecté.
